(19) Europäisches Patentamt
European Patent Office
Office européen des brevets

(11) **EP 0 980 241 B1**

(12) **EUROPEAN PATENT SPECIFICATION**

(45) Date of publication and mention
of the grant of the patent:
**17.12.2003 Bulletin 2003/51**

(51) Int Cl.[7]: **A61K 9/48**, A61K 31/135

(21) Application number: **98923021.4**

(86) International application number:
**PCT/IB98/00936**

(22) Date of filing: **16.06.1998**

(87) International publication number:
**WO 99/001113 (14.01.1999 Gazette 1999/02)**

(54) **GELATINE ENCAPSULATED SOLUTION DOSAGE FORMS OF SERTRALINE**

IN GELATINE EINGEKAPSELTE SERTARLINE DOSIERUNGSFORMEN

FORMES POSOLOGIQUES DE SERTRALINE, EN SOLUTION ET ENCAPSULEES DANS DE LA GELATINE

(84) Designated Contracting States:
**AT BE CH CY DE DK ES FI FR GB GR IE IT LI LU NL PT SE**
Designated Extension States:
**AL LT LV MK RO SI**

(30) Priority: **01.07.1997 US 51401 P**

(43) Date of publication of application:
**23.02.2000 Bulletin 2000/08**

(73) Proprietor: **Pfizer Products Inc.**
**Groton, Connecticut 06340 (US)**

(72) Inventors:
• **CURATOLO, William, John**
**Niantic, CT 06357 (US)**
• **SHANKER, Ravi, Mysore**
**Groton, CT 06340 (US)**

(74) Representative: **Wood, David John et al**
**PFIZER LIMITED,**
**Ramsgate Road**
**Sandwich, Kent CT13 9NJ (GB)**

(56) References cited:
**EP-A- 0 768 083**       **WO-A-96/09047**

**Description**

This invention relates to an encapsulated solution dosage form of sertraline which exhibits, relative to conventional immediate-release dosage forms, a shorter time to reaching peak blood levels after oral dosing, and which also exhibits reduced side effects. The invention further relates to a method of treating psychiatric and other illnesses comprising administering sertraline in such a dosage form to a mammal, including a human patient, in need of such treatment.

Background of the Invention

Sertraline is a selective serotonin reuptake inhibitor (SSRI), which is useful as an antidepressant and anorectic agent, and in the treatment of obsessive-compulsive disorder, post-traumatic stress disorder, anxiety-related disorders and panic. Sertraline is also useful for the treatment of premature ejaculation, chemical dependencies, premenstrual dysphoric disorder, and obesity.

EP-A-768083 and WO-A-9 609 047 disclose certain sertraline compositions.

Sertraline is most commonly prescribed for therapy of depressive illness in the general dose range 50-200 mg/day. Sertraline has an elimination half-life of 23 hr, and is dosed once daily. The absolute oral bioavailability of sertraline dosed as Zoloft® tablets is 54%. Thus sertraline is a well-absorbed drug, and there is no motivation generally to try to improve the oral absorption of sertraline.

In the case of known, oral immediate-release dosage forms of sertraline, it is known that $T_{max}$, the time at which a maximal plasma sertraline concentration is achieved, is approximately 6-7 hours. Generally speaking, such a several hour duration constitutes a long $T_{max}$. It would be useful to have dosage forms available which would deliver sertraline in a manner which effects a $T_{max}$ that is less than the $T_{max}$ of 7 hr typically observed for currently available immediate-release oral dosage forms. Lessening $T_{max}$ may result in a decreased onset of antidepressive action. A shortened $T_{max}$ would be particularly useful in acute indications such as panic and premature ejaculation, and could also be useful in more chronic indications such as depression.

Patients are generally initiated on sertraline for depression at a dose of 50 mg/day. Patients who do not respond at the 50 mg dose are given higher doses. Initiation at doses greater than 50 mg is generally avoided, when possible, because side effects such as nausea, diarrhea and regurgitation are generally believed to be more severe at higher doses. If necessary, for example because toleration to sertraline side effects has occurred in a patient, higher doses may be reached by slow titration up from lower doses.

A dosage form which resulted in a shorter $T_{max}$ could have improved efficacy, particularly in acute indications, and thus such a dosage form may provide the additional advantage of being efficacious at a lower dose than a dosage form which results in a longer Tmax. By dosing lower doses, certain side effects may be ameliorated, e.g. gastrointestinal side effects which are partially or primarily mediated by local contact of sertraline with the walls of the gastrointestinal tract.

Thus, an improved sertraline dosage form which permitted oral dosing of sertraline with relatively reduced side effects and a shortened $T_{max}$ would permit wider therapeutic application of sertraline therapy, and would accordingly provide a significant improvement in dosing compliance and convenience.

Formulations of drugs in solution in soft gelatin or hard gelatin capsules are known and are well understood in the art. These dosage forms comprise a water-soluble soft or hard gelatin exterior shell which encapsulates a vehicle in which a drug has been dissolved and/or suspended. The disclosure which follows refers for convenience to "soft-gels" as an abbreviation for soft gelatin capsules. It is understood that the discussion applies equally to all types of gelatin capsules, regardless of hardness, softness, and so forth.

It has been determined that the capacity of the small intestine to absorb sertraline is high. In a human duodenal infusion, it was determined that the sertraline intrinsic absorption rate constant (ARC) is greater than 0.025 min$^{-1}$. A half-time for absorption may be calculated as 0.693/ARC, giving a value of less than 27.7 minutes. Since the majority of the absorptive process would be over by 3 absorption half-times, the theoretical $T_{max}$ for intestinally dosed sertraline is less than 83 minutes. Allowing for gastric emptying of an orally dosed solution, the $T_{max}$ for orally dosed sertraline should be under 1.5 hr. Thus sertraline should exhibit a very short $T_{max}$.

Furthermore, it has been determined that dosing an aqueous solution of sertraline directly to the stomach of humans results in a $T_{max}$ of 7 hr, as demonstrated in Example 1. This result would lead one to conclude that oral dosing of soft-gel-encapsutated solutions of sertraline would not have the effect of decreasing $T_{max}$, relative to currently known solid tablet dosage forms. Furthermore, in Example 1, it is demonstrated that direct delivery of an aqueous sertraline solution to the duodenum results in a $T_{max}$ of 3.7 hr, which is about 1/2 the $T_{max}$ value observed after gastric solution dosing (7 hr), but still much longer than the theoretical $T_{max}$ estimated from 0.693/ARC.

Very few drugs are formulated as solutions in gelatin capsules due to solubility constraints. A drug, in order to be a candidate for soft-gel formulation, needs to be highly soluble and chemically stable in the soft-gel vehicle employed. The most common soft-gel solvents or vehicles are water-immiscible triglyceride vegetable oils such as sesame oil, corn oil, and olive oil, water-immiscible refined and synthetic and semisynthetic oils such as the triglycerides known as Miglyols®, the water-miscible alcohols glycerin and propyleneglycol, and water-miscible polyethyleneglycols (PEGs) which are liquid at room temperature, such as PEG-400. The choice of a soft-gel vehicle for a particular drug is generally based on achieving dissolution of the therapeutic dose in a volume of the vehicle which will fit in a reasonably sized soft-gelatin capsule (e.g. in 0.8 ml or less). For example, for a 50 mg dose of a drug to fit in a 0.8 ml soft-gel it would require a solubility of at least 50 mg/0.8 ml or 62.5 mg/ml. It is not practical, however, to place a drug in solution in a soft-gel at the drug's saturation solubility because the soft-gelatin shell contains water which can partition into the vehicle, resulting in precipitation of the drug, generally observed as crystallization of drug on the inner surface of the capsule shell. Thus, as a general rule it is desirable to keep the dissolved drug at approximately 75% of saturation (or less) in the solution within the soft-gel capsule. The 50 mg dose discussed above should accordingly have a solubility in the soft-gel vehicle of at least 62.5 mg/ml divided by 0.75, or 83 mg/ml. This solubility constraint is a severe one which is rarefy met, hence a reason that very few drugs are formulated as solutions in gelatin capsules.

Summary of the Invention

This invention provides encapsulated solution dosage forms of sertraline (1) which exhibit a $T_{max}$ which is decreased relative to the $T_{max}$ exhibited by currently known immediate release sertraline tablet dosage forms which deliver an equivalent bolus dose, (2) and/or which decrease one or more gastrointestinal side effects of sertraline, and/or (3) which decrease, relative to a comparison dosage form made with a water-miscible vehicle, precipitation of sertraline in a chloride ion containing use environment, such as the stomach, small intestine, or *in vitro* test fluid which simulates such an environment.

More specifically, this invention provides a dosage form comprising a gelatin-encapsulated composition comprising sertraline, or a pharmaceutically acceptable salt thereof, in a water-immiscible vehicle. The vehicle is preferably liquid at room temperature, although semi-solid vehicles which are liquid or contain liquid phases at mammalian body temperature, e.g. 36 - 38°C, are also acceptable. Preferred vehicles of this invention possess a sertraline solubility which permits the desired therapeutic dosage to be dissolved at 75% saturation in 0.8 ml, at room temperature. As described above, for a dosage of 50 mg, a preferred vehicle will have a sertraline solubility of at least 83 mg/ml. Likewise, for a 10 mg or 100 mg dose, a more preferred vehicle will have a sertraline solubility of at least 16.7 mgA/ml or at least 167 mgA/ml, respectively. Thus, preferred vehicles are those water-immiscible vehicles in which sertraline, or one of its pharmaceutically acceptable salts, is soluble enough to provide a dose of 10mgA or greater, at 75% saturation, in 0.8ml of solvent. Thus preferred vehicles exhibit a sertraline solubility of about 16.7 mgA/ml or greater. Also useful in this invention are vehicles in which sertraline forms a suspension, as long as 50% of the incorporated sertraline is in solution in the vehicle at mammalian body temperature.

The invention is surprising because encapsulated solution dosage forms are typically formulated for drugs and vitamins which have extremely low aqueous solubility, e.g. cyclosporin (6 microgm/ml at 37°C) and vitamin E (practically insoluble in water). It is therefore surprising that encapsulated solutions of sertraline would be of value because sertraline is considered to be a water-soluble compound. The solubility of sertraline hydrochloride at the pH of the stomach is approximately 3 mg/ml. This is an excellent aqueous drug solubility which would generally be more than sufficient to support dissolution and absorption of drugs at doses of hundreds of milligrams (see Johnson and Swindell (1996) Pharmaceutical Research 13, 1795-1798, for an analysis of aqueous solubility requirements for drug absorption). Furthermore, the aspartate, acetate, and lactate salts of sertraline have higher solubilities in water, with the lactate having the highest solubility of these (125 mg/ml). Thus there would be no inclination to prepare soft-gelatin formulations of sertraline or its salts, because sertraline is not a low solubility drug.

It has been determined that solutions of certain sertraline salts can form poorly soluble gels in the presence of chloride ion. For example, a saturated aqueous solution of sertraline lactate (125 mg/ml) forms a gel when the chloride ion concentration exceeds about 0.06M. At physiological chloride concentrations (0.15M) a saturated solution of sertraline lactate or sertraline acetate becomes a thick pasty solid which does not flow. The observation of chloride-induced sertraline gelling was unexpected.

Furthermore, sertraline is a base which has a lower solubility at intestinal pH than at gastric pH. In addition, the solubility of sertraline is chloride-ion dependent, with lower observed solubility in the presence of chloride. The unexpected formation of sertraline gels is believed to be facilitated when the aqueous sertraline concentration is high, e.g. when the high solubility aspartate, acetate, and lactate salts are dissolved. While not wishing to be bound by theory, presented anywhere herein, it is believed that the soft-gel formulations may shorten sertraline $T_{max}$ by interfering with the chloride-induced gelling of sertraline *in vivo* or by interfering with precipitation of low solubility sertraline salts at intestinal pH. For example, the soft-gel vehicle may act by sequestering sertraline from the chloride ion-containing environment of the GI tract until it reaches the small intestine where it is released and rapidly absorbed, thereby shortening $T_{max}$.

A dosage form according to the invention is thus a hard-gel or soft-gel capsule containing a solution or suspension comprising sertraline, or a pharmaceutically acceptable sertraline salt, dissolved in a water-immiscible vehicle.

"Water-immiscible" means that the vehicle forms a separate phase when added to an aqueous environment. The particular water-immiscible phase formed by the vehicle or the name used to identify the phase is not believed to be particularly important The phase may be an emulsion, a microemulsion, or it may otherwise form phase-separated droplets within the aqueous environment of the gastrointestinal tract.

As further discussed and disclosed below, the vehicle may also form micelles in an aqueous environment Micelles, by virtue of having a polar exterior and a non-polar interior, form micellar solutions and are technically not generally described as water-immiscible. However, while not wishing to be bound by theory, it is believed that the non-polar hydrophobic interior of a micelle acts to sequester sertraline in the aqueous GI tract and that because of their hydrophobic interior, micelles are operatively equivalent or functionally similar to otherwise phase-separated, water-immiscible vehicles for purposes of this invention. Thus any vehicle which forms micelles in an aqueous environment is considered to be "water-immiscible" for purposes of this invention.

The term "ingestion" as used herein is essentially synonymous with "swallowing".

The amount of sertraline encapsulated as a solution or suspension in a hard or soft gelatin capsule is preferably at least 10 mgA, and can be as high as 300 mgA or more. The amount contained in the dosage form is preferably 10 mgA to 250 mgA, more preferably 10mgA to 100mgA. The dosage form can be unitary, or divided e.g., constituted by two or more units (such as capsules which, taken together, constitute the dosage form) which are taken at or about the same time.

Sertraline can be employed in the dosage forms of this invention in the form of its base or pharmaceutically acceptable salts, and also in anhydrous as well as hydrated forms. All such forms can be used within the scope of this invention. The sertraline employed is preferably the free base, hydrochloride, aspartate, acetate, or lactate. The latter three salts are disclosed in EP-A-0 999 830.

Reference to "sertraline" in terms of therapeutic amounts in the claims is to active sertraline, abbreviated herein as "mgA", i.e., active sertraline, the non-salt, non-hydrated free base having a molecular weight of 306.2. "mgA" can easily be converted to an equivalent weight for whatever salt or hydrate form is desired.

In a further aspect, this invention provides a method of treating an illness amenable to treatment with sertraline, comprising administering, to a person in need of such treatment, a dosage form comprising an encapsulated solution or suspension of a therapeutically effective amount of sertraline, or a pharmaceutically acceptable salt thereof, in a water immiscible vehicle. Such illnesses include those known in the art as being treatable with sertraline, including those mentioned above.

It is an object of this invention to provide a dosage form of sertraline which has a shorter $T_{max}$ than conventional sertraline dosage forms, thus permitting faster appearance of sertraline in the bloodstream, and a potentially faster therapeutic effect. A faster therapeutic effect is of particular importance in acute indications such as the amelioration of panic or premature ejaculation.

It is a further object of this invention to provide a dosage form of sertraline which decreases precipitation of sertraline in a chloride-ion containing use environment such as the stomach, small intestine, or *in vitro* simulated chloride ion-containing gastric fluids relative to a dosage form identical thereto except it contains a water-miscible vehicle instead of a water-immiscible vehicle.

Detailed Discussion

The required solubility of sertraline or of the particular pharmaceutically acceptable salt in the particular vehicle employed depends on the quantity of sertraline required for the softgel. Capsules of this invention typically have a volume of 1.5 ml or less. Preferred capsules of this invention have a volume of 1 ml or less. More preferred capsules have a volume of 0.8 ml or less. More than one capsule may be dosed concurrently to achieve a therapeutic dose.

A dosage form of the invention, in its simplest form, can be prepared by dissolving a therapeutic amount of sertraline base or one of its salts, preferably the hydrochloride, aspartate, acetate, or lactate salt, in an appropriate vehicle, and encapsulating the solution in a soft or hard gelatin capsule by conventional methodology. Sertraline vehicles of this invention comprise solvents or mixtures of solvents which form immiscible droplets, microemulsions, or micelles when added to water. Single solvent vehicles in which the solvent dissolves in water as molecular monomers are not useful as vehicles in this invention. Thus single water-miscible solvents such as ethanol or PEG-400, which dissolve molecularly in water, are not useful. However, such water-miscible solvents may be contained in the sertraline vehicle (e.g. to improve sertraline solubility) as minor components, i.e. at less than about 30% of the total vehicle volume.

Water-immiscible solvents of this invention include water-immiscible oils, including triglyceride vegetable oils such as safflower oil, sesame oil, olive oil, com oil, castor oil, coconut oil, cottonseed oil, soybean oil, and the like. Also included are synthetic and semisynthetic medium chain triglyceride oils such as those sold under the trademark Miglyol® (HulsAmerica, Piscataway, New Jersey) or Captex® (Abitec Corp., Columbus, Ohio). Examples are triglycerides of caprylic/capric acids (Miglyol®-810, Miglyol®-812, Captex®-300, Captex®-355), and triglycerides of caprylic/capric/linoleic acids (Miglyol®-818). Also included are long chain triglyceride oils such as triolein, and other mixed chain triglycerides which are liquid at room temperature. Also included is mineral oil.

Water-immiscible solvents also include monoglycerides and diglycerides such as those sold under the trademarks Capmul® (Abitec Corp., Columbus, Ohio) and Imwitor® (HulsAmerica, Piscataway, New Jersey). Examples are monoolein (Capmul®-GMO), mono and diglycerides of octanoic and decanoic acids (Imwitor®-742, Capmul®-MCM), and monooctanoin (Imwitor®-308), and the like.

Mixtures of mono-, di, and/or triglycerides can also be used.

Preferred oils are liquid at room temperature. Preferred mono-, di-, and triglycerides are those with an average acyl chain length of 4-18 carbons, more preferably 6-14 carbons.

Useful vehicles further include various liquid esters of short chain acids and alcohols, such as the propylene glycol ester of caprylic and/or capric acids (Miglyol®-840, Captex®-200). Fatty acids which are liquid at room or body temperature, such as caprylic acid, capric acid, lauric acid, oleic acid, or linoleic acid are also useful.

Further useful vehicles include semisolid vehicles such as those sold under the tradename Gelucire®. Examples are PEG-32-glyceryl-laurate (Gelucire®44/14), and glycerol esters of fatty acids (Gelucire®33/01).

Further useful vehicles also include surfactants and emulsifiers which have the capacity to dissolve sertraline. These surfactants and emulsifiers form micelles when they are mixed with aqueous media. Examples are polysorbate-80, nonylphenoxypolyoxethylenes, dioctyl sodium sulfosuccinate, PEG-6 glyceryl mono-oleate (Labrafil® M-1944-CS), PEG-6 glyceryl linoleate (Labrafil® M-2125-CS), and the like.

As described above, preferred vehicles are those which can dissolve sertraline or any one of its pharmaceutically acceptable salts at a concentration of about 16.7 mgA/ml or greater. As illustrated in Example 4 below, certain encapsulation vehicles have a higher capacity than others for maintaining sertraline in solution after the formulation has mixed with a chloride ion-containing environment such as simulated gastrointestinal contents. More preferred vehicles are those which inhibit precipitation of sertratine in the presence of either 0.1N HCl or phosphate buffered saline, pH 5.8, determined as described in Example 4. These encapsulation vehicles are more preferred because they minimize precipitation or gelling of sertraline in the use environment, i.e. the gastrointestinal lumen, thus maximizing the speed with which sertraline can appear in the bloodstream after dosing. Even if these preferred vehicles do not completely or almost completely prevent the precipitation of sertraline when mixed with chloride-containing model physiological fluids, any effect on sertraline precipitation rate is advantageous. In vivo, the intestinal wall has a high capacity for rapidly absorbing sertraline, revealed by a high absorption rate constant (ARC). Any formulation which helps keep sertraline in solution, even temporarily, Will be useful because precipitation and absorption compete for the available soluble sertraline.

More preferred vehicles, according to this criterion, are vegetable oils such as safflower oil and olive oil; medium chain triglycerides such as caprylic/capric triglycerides; mono- and di-glycerides including medium chain mono- and di-glycerides; acylated polyols such as propylene glycol dicaprylate/caprate; fatty acids such as oleic acid; and surfactants such as polysorbate-80.

Most preferred vehicles, as illustrated in Example 4, are those which inhibit sertraline precipitation in 0.1 N HCl and in phosphate buffered saline, pH 5.8. These include medium chain (i.e., 6-14 carbons per chain) triglycerides such as caprylic/capric triglycerides; mono- and di-glycerides including medium chain mono- and di-glycerides; acylated polyols such as propylene glycol dicaprylate/caprate; fatty acids such as oleic acid; and surfactants such as polysorbate-80. Most preferred vehicles have the capacity to solubilize sertraline hydrochloride in the use environment, thus minimizing the precipitation of this salt in chloride-containing physiological solutions, regardless of whether sertraline has been originally dosed as free base, hydrochloride salt, or other pharmaceutically acceptable salt. Most preferred vehicles exhibit a sertratine hydrochloride salt solubility greater than about 0.1 mgA/ml (to inhibit sertraline precipitation in physiological fluids), in addition to exhibiting a sertraline solubility greater than about 16.7 mgA/ml for any form of sertraline (to permit dosing 10 mgA or more in a 0.8 ml gelatin capsule).

Thus, vehicles useful in this invention are water immiscible, and may be generally ordered in increasing order of preference as follows:

1. Useful - Water immiscible vehicles;

2. Preferred - Category (1) Vehicles in which any form of sertraline (i.e. the free base or any acid addition salt) exhibits a solubility of at least 16.7 mgA/ml

3. More preferred - Category (2) vehicles which, relative to water-miscible vehicles, decrease precipitation of any form of sertraline in a chloride-ion containing simulated gastric fluid such as 0.1N HCl or in a chloride-ion containing simulated intestinal fluid such as 50mM phosphate buffer, pH 5.8, containing 0.15 M sodium chloride

4. More preferred - Category (2) vehicles which, relative to water-miscible vehicles, decrease precipitation of any form of sertraline in a chloride-ion containing simulated gastric fluid such as 0.1N HCl and in a chloride-ion containing simulated intestinal fluid such as 50mM phosphate buffer, pH 5.8, containing 0.15 M sodium chloride

Water-immiscible solvents may be mixed with surfactants and emulsifiers, in order to effect the spontaneous formation of small or microscopic vehicle droplets (e.g. microemulsions) when the water-immiscible solvent/emulsifier vehicle is mixed with water, as in the gastrointestinal tract. Such mixtures include mixtures of triglycerides, or mono- and di-glycerides, with polysorbates, e.g. mixtures of Capmul®-MCM and polysorbate-80, or mixtures of Miglyol® -812 and polysorbate-80, in ratios of from 99/1 to 50/50, respectively. Further useful mixtures include mixtures of mono-, di-, and triglycerides with polysorbates, e.g. Capmul®-MCM/Miglyol®-812/polysorbate-80, in which Capmul®-MCM makes up 40-80% of the vehicle, with the remainder being any combination of Miglyol-812 and polysorbate-80. Further useful mixtures include a vegetable oil and a surfactant, e.g. olive oil/polysorbate-80 in ratios of 99:1 to 50:50, or corn oil/Labrafil®-M-2125-CS in ratios of 99:1 to 50:50. Polyethyleneglycols (typically of average molecular weight 200-600) and other water-miscible solvents such as glycerin, ethanol, propylene glycol, may be included in amounts up to 30% of the vehicle, in order to optimize sertraline solubility in the vehicle, or to improve the viscosity of the vehicle to aid in capsule filling.

Solutions of sertraline in vehicles of the types described above are encapsulated in soft gelatin capsules, or are encapsulated in hard gelatin capsules. If encapsulated in hard gelatin capsules, it is preferred that the seam between the two capsule shell pieces be sealed, for example with a strip of gelatin, to prevent leakage. Encapsulation in soft-gelatin is well-known , and is described in "The Theory and Practice of Industrial Pharmacy", by L. Lachman, H. Lieberman, and J. Kanig, Lea and Febiger, publisher.

Dosage forms of this invention, upon oral dosing, result in a decrease in $T_{max}$ of at least 0.5 hr, preferably at least 1 hour, more preferably at least 1.5 hr relative to an immediate release sertraline tablet, e.g. a Zoloft® tablet. To test whether a dosage form decreases $T_{max}$, a cross-over clinical study can be carried out in 12 or more healthy fasted human volunteers. One half of the group receives the test sertraline dosage form and one half of the group receives an immediate release sertraline dosage form (e.g. Zoloft® tablets) at the same dose. Blood is collected at appropriate times before- and post-dose, and the blood sertraline concentration is determined by an appropriate assay, as described in the examples below. After a wash-out period of at least one week, each group receives the alternate dosage form, and blood sertraline concentrations are determined as before. $T_{max}$ (for the immediate release dosage form) minus $T_{max}$ (for the test dosage form) is determined for each subject. These differences are then averaged to give an average $T_{max}$ difference. If this value is greater than 0.5 hr, then the dosage form is a dosage form of this invention. If this value is greater than 1 hr, then the dosage form is a preferred dosage form of this invention.

For clarification, the following information is provided:

1. Specification of a quantity in percent (%) means percent by weight based on total weight, unless otherwise indicated.
2. "Use environment" means the aqueous environment of the gastrointestinal tract.
3. "mgA" refers to mg active sertraline, equivalent to the free base.

Example 1

This example demonstrates that the absorption of sertraline differs when sertraline is dosed directly to various portions of the gastrointestinal tract. In particular, this example demonstrates that delivery of sertraline directly to the duodenum results in more rapid achievement of peak sertraline plasma levels, compared with the more usual oral delivery to the stomach. This indicates that $T_{max}$ can be decreased by altering the sertraline formulation, and is not limited to 6-7 hr by post-absorptive metabolic phenomena. This example further demonstrates that delivery of an aqueous solution of sertraline directly to the stomach does not result in a decrease in $T_{max}$ relative to dosing an immediate release tablet. Thus oral delivery of sertraline in an encapsulated solution which is water-miscible will not result in a decrease in $T_{max}$, relative to currently known immediate release sertraline tablets.

Two groups of 6 volunteers each were dosed with 200 mg sertraline or placebo by different four-way crossover regimens. Dosing was via (1) oral tablets, or (2) infusion of a solution through a nasoenteric tube into the stomach, duodenum, or ileocecal region of the small intestine, or (3) infusion into the transverse colon via anal intubation.

On four different occasions, Group A received (1) oral sertraline immediate release tablets plus placebo solution infused into the stomach, or (2) oral placebo tablets plus sertraline solution infused into the stomach, or (3) oral placebo tablets plus sertraline infused into the small intestine at the ileocecal junction, or (4) oral placebo tablets plus placebo solution infused into the small intestine at the ileocecal junction. On four different occasions, Group B received (1) oral sertraline immediate release tablets plus placebo solution infused into the duodenum, or (2) oral placebo tablets plus sertraline solution infused into the duodenum, or (3) oral placebo tablets plus sertraline infused into the transverse colon, or (4) oral placebo tablets plus placebo solution infused into the transverse colon.

The oral sertraline dose was administered as two 100 mg Zoloft® tablets. The infusions were administered as a 2 mg/ml solution at a rate of 20 ml/min for 5 min.

Blood samples were withdrawn prior to dosing, and at 0.5, 1, 1.5, 2, 4, 6, 8, 10, 12, 16, 24, 36, 48, 72, 96, 120, 144, 192, and 240 hr post-dosing. Plasma sertraline concentrations were determined by extraction of sertraline from basic human plasma into methyl t-butyl ether, followed by derivatization to form the trifluoroacetyl adduct. Analysis was carried out by capillary gas chromatography with electron capture detection. Total systemic exposure to sertraline was determined by measuring the area under the plasma sertraline concentration vs. time curve (AUC) for each subject in a given group, and then by calculating a mean AUC for the group. $C_{max}$ is the highest plasma sertraline concentration achieved in a subject. $T_{max}$ is the time at which $C_{max}$ is achieved. Plasma pharmacokinetic data for this example are presented in Table 1.

Table 1-1 presents the observed average $C_{max}$, $T_{max}$, and AUC for the various dosing regimens. Infusion into the stomach gave $C_{max}$. $T_{max}$, and AUC values which were similar to those observed after oral dosing of tablets (Group A). This indicates that the technique of infusion does not in itself cause any substantive change in the pharmacokinetics of sertraline. Furthermore, it indicates that the time required for disintegration and dissolution of sertraline tablets is not a factor in causing the long (7 hr) $T_{max}$. infusion into the duodenum gave $C_{max}$ and AUC values which were similar to those observed after oral dosing of tablets. However, infusion into the duodenum (Group B) gave a $T_{max}$ which was surprisingly shorter than that observed after oral dosing of tablets or gastric infusion of an aqueous sertraline solution. This indicates that alteration of the method of oral drug delivery can result In a desired decrease in $T_{max}$.

This example suggests that sequestering a portion of an oral sertraline dose until it enters the duodenum can result in a decreased $T_{max}$. It also demonstrates that The $T_{max}$ observed on direct duodenal dosing (3.7 hr) is longer than the $T_{max}$ theoretically possible for a drug with an intestinal permeability as high as that exhibited by sertraline (see disclosure).

Table 1-1

| Pharmacokinetics of 200mg sertraline delivered to various portions of the gastrointestinal tract. | | | |
|---|---|---|---|
| **GROUP A** | | | |
| **Dosing Route** | **$C_{MAX}$ (ng/ml)** | **$T_{MAX}$ (hr)** | **$AUC_{o-LAST}$(ng·hr/ml)** |
| Oral Tablet | 39.9 | 7.0 | 1174.5 |
| Stomach Infusion | 35.6 | 7.0 | 923.1 |
| Ileocecal Infusion | 27.3 | 5.0 | 727.1 |
| **GROUP B** | | | |
| **Dosing Route** | **$C_{MAX}$ (ng/ml)** | **$T_{MAX}$ (hr)** | **$AUC_{o-LAST}$(ng ·hr/ml)** |
| Oral Tablet | 44.7 | 6.7 | 1153.4 |
| Duodenal Infusion | 48.8 | 3.7 | 1270.3 |
| Colonic Infusion | 10.9 | 4.4 | 179.4 |

Example 2

The solubilities of sertraline free base, sertraline hydrochloride, and sertraline lactate were determined in a series of solvents as follows. Seventy-five mg sertraline base or salt was weighed into a centrifuge tube, and 250 microgm solvent was added. If the entire 75 mg did not dissolve, solvent was added in 250 microgm increments until the sertraline dissolved. The concentration of dissolved sertraline was determined by HPLC. Dissolved sertraline samples were also stored at 5 degC overnight, then warmed to room temperature. All dissolved samples remained in solution after this temperature treatment. Determined solubilities are reported in Table 2-1.

As described in "BACKGROUND OF THE INVENTION", preferred vehicles for a 50 mgA dose exhibit a sertraline solubility greater than 83 mg/ml. Thus, for a dose of 50mgA, useful sertraline/vehicle combinations are sertraline lactate in Capmul®-MCM, and sertraline base in Capmul®-MCM, safflower oil, or polysorbate-80. Preferred vehicles for a 10mgA dose exhibit a sertraline solubility greater than 16.7 mgA/ml. Thus for a dose of 10 mgA, useful sertraline/vehicle combinations are sertraline lactate or hydrochloride in Capmul®-MCM, and sertraline base in Capmul®-MCM, safflower oil, or polysorbate-80. PEG-400 is not a sertraline vehicle useful in this invention because it is miscible with water.

Table 2-1

| Solubilities of Sertraline (as Base or Hydrochloride or Lactate Salt) in Selected Vehicles. | | | | |
|---|---|---|---|---|
| Salt or Base | Solubility in PEG-400 (mga/ml) | Solubility in Capmul-MCM (mgA/ml) | Solubility in Safflower Oil (mgA/ml) | Solubility in Polysorbate-80 (mgA/ml) |
| Free base | 286<X<572 | 150<X<300 | 200<X<400 | ~102 |
| Hydrochloride | 13<X<18 | ~45 | <9 | <14 |
| Lactate | ~23 | 97<X<193 | <8 | <12 |

Example 3

This example demonstrates that polysorbate-80 is a sertraline vehicle which prevents precipitation of sertraline in the presence of chloride ion. Sertraline base was dissolved in PEG-400 or polysorbate-80. One ml of this test solution was added to 15 ml normal saline (0.9% NaCl, pH 4.2), and was stirred vigorously. After 15 min and 2 hr, aliquots were taken, filtered, and assayed for sertraline by HPLC. Table 3-1 demonstrates that upon addition of a PEG-400 solution of sertraline to a NaCl solution, a portion of the sertraline precipitates. When a Polysorbate-80 solution of sertraline is added to a NaCl solution, no precipitation occurs.

Table 3-1

| Dilution of PEG-400 or Polysorbate-80 Solution of Sertraline Base into 0.9% NaCl. | | | | |
|---|---|---|---|---|
| Vehicle | Concentration in Vehicle (mgA/ml) | Expected Concentration after Dilution in Saline (mgA/ml) | Observed Concentration after Dilution in Saline, after 15 min (mgA/ml) | Observed Concentration after Dilution in Saline, after 2 hr (mgA/ml) |
| PEG-400 | 50 | 3.1 | 0.9 | 2.1 |
| PEG-400 | 100 | 6.25 | 0.7 | 1.8 |
| Polysorbate-80 | 50 | 3.1 | 3.1 | 3.1 |
| Polysorbate-80 | 100 | 6.25 | 6.25 | 6.25 |

Example 4

This example illustrates an *in vitro* test methodology for the selection of more preferred vehicles for sertraline. As described previously, preferred vehicles are those water-immiscible vehicles which can dissolve sertraline or one of its salts at a concentration sufficient to permit encapsulation of a therapeutic dose in a capsule of a size which can be easily swallowed. As illustrated in this example, more preferred vehicles are those that decrease the precipitation of sertraline in the presence of (a) 0.1 N hydrochloric acid and/or (b) 50 mM phosphate buffer at pH 5.8 containing 0.15M sodium chloride. Solution (a) is a simulated gastric fluid, and solution (b) is a simulated intestinal fluid. Rapid absorption of sertraline is facilitated in vivo by a decreased rate of precipitation of low solubility sertraline salts and by a decreased rate of formation of low solubility sertraline gels.

Sertraline base was dissolved in the following three vehicles at a concentration of 50 mg/mL: (1) Polyethylene glycol-400 (PEG-400), (2) Capmul®-MCM and (3) Safflower oil. PEG-400 is miscible with aqueous media, while Capmul® -MCM and safflower oil are not. One milliliter of the test solution was added to 10 mL of (a) 0.1 N HCl solution and (b) phosphate buffered saline (PBS) pH 5.8 and was stirred vigorously. After 15 minutes and 2 hours, aliquots of the sample were taken, and were centrifuged to separate solids as well as the aqueous and non-aqueous layers. Aliquots of the aqueous and non-aqueous layers were taken, filtered and assayed for sertraline by HPLC. The experimental design is schematized in Table 4-1. Tables 4-2 and 4-3 summarize the results from these investigations. The results indicate that the concentrations of sertraline at 15 minutes and 2 hours are not Significantly different from each other. Data in these Tables demonstrate that addition of a PEG 400 solution of sertraline into either 0.1 N HCl or phosphate buffered saline results in the precipitation of a large proportion of sertraline. In the case of Capmul®-MCM the data demonstrate that a major portion (>95% of added amount) of sertraline remains in solution. Consequently there is essentially no precipitation of sertraline. In the case of safflower oil, in the presence of 0.1N HCl there is significant but incomplete (81%) precipitation of sertraline. However, little or no precipitation is observed when the solution of sertraline base in safflower oil is added to phosphate buffered saline.

These results demonstrate that water-miscible sertraline vehicles such as PEG-400 do not have the capacity to maintain sertraline in solution under physiological conditions. Water-immiscible vehicles do have this capacity. Capmul®-MCM (mono- and di-glycerides of octanoic and decanoic acids) has the capacity to maintain sertraline in solution in the presence of simulated gastric fluid and in the presence of simulated intestinal fluid. Thus medium chain mono- and di-glycerides are members of a most preferred group of sertraline encapsulation solvents. While not wishing to be bound by theory, it is likely that this most preferred group possesses the capacity to solubilize the hydrochloride salt of sertraline, thus maintaining sertraline solubility in the presence of chloride-containing simulated gastric fluid or simulated intestinal fluid, regardless of the form of sertraline originally dosed. Table 4-4 presents the equilibrium solubility of sertraline hydrochloride in a variety of water-immiscible sertraline solvents suitable for use in encapsulated sertraline solution dosage forms. Table 4-4 demonstrates that Capmul®-MCM, Miglyol®-810 (caprylic/capric triglycerides), Captex®-200 (propylene glycol dicaprylatelcaprate), and oleic acid have the capacity to dissolve sertraline hydrochloride at greater than 0.1 mgA/ml; olive oil and safflower oil do not.

# Table 4-1: Flow chart of the precipitation studies with sertraline base

EP 0 980 241 B1

Table 4-2:

| Results from HPLC analysis for sertraline in the precipitation studies: 15 minutes after addition of sertraline free base solution into the dissolution media. | | | | |
|---|---|---|---|---|
| Initial concentration of sertraline free base in | Dissolution media (10 mL) | Concentration of sertraline in aqueous layer (mgA/mL) | Concentration of sertraline in non-aqueous layer (mgA/ mL) | Expected concentration of sertraline in aqueous layer (mgA/mL) |
| PEG 400 50 mgA/mL (1 mL added to 10 mL of dissolution media) | 0.1 N HCl | 0.6 | XX | 4.55 |
| | Phosphate buffered saline pH 5.8 | 0.16 | XX | 4.55 |
| Capmul®MCM 50 mgA/ mL (1 mL added to 10 mL of dissolution media) | 0.1 N HCl | 0.38 | 44 | 5.00 |
| | Phosphate buffered saline pH 5.8 | 0.18 | 48 | 5.00 |
| Safflower oil 50 mgA/ mL (1 mL added to 10 mL of dissolution media) | 0.1 N HCl | 0.48 | 4.8 | 5.00 |
| | Phosphate buffered saline pH 5.8 | 0.19 | 45 | 5.00 |

Table 4-3:

| Results from HPLC analysis for sertraline in the precipitation studies: 120 minutes after addition of sertraline free base solution into the dissolution media. | | | | |
|---|---|---|---|---|
| Initial concentration of sertraline free base in | Dissolution media (10 mL) | Concentration of sertraline in aqueous layer (mgA/ mL) | Concentration of sertraline in non-aqueous layer (mgA/ mL) | Expected concentration of sertraline in aqueous layer (mgA/ mL) |
| PEG 400 50 mgA/mL (1 mL added to dissolution media) | 0.1 N HCl | 0.35 | XX | 4.55 |
| | Phosphate buffered saline pH 5.8 | 0.12 | XX | 4.55 |
| Capmul®MCM 50 mgA/ mL (1 mL added to dissolution media) | 0.1 N HCl | 0.31 | 44.2 | 5.00 |
| | Phosphate buffered saline pH 5.8 | 0.16 | 48.6 | 5.00 |
| Safflower oil 50 mgA/ mL (1 mL added to dissolution media) | 0.1 N HCl | 0.48 | 3.2 | 5.00 |
| | Phosphate buffered saline pH 5.8 | 0.19 | 44.6 | 5.00 |

Table 4-4:

| Equilibrium solubility of sertraline base and hydrochloride in selected vehicles | | |
|---|---|---|
| **Vehicle** | **Free base sol. in mgA/ml** | **Hydrochloride Sol in mgA/mL** |
| Capmul-®MCM | >50 | 38.0 |
| Migloyol® 810 | >50 | 42.0 |
| Captex® 200 | >50 | 0.63 |
| Oleic acid | >50 | 0.82 |

Table 4-4:   (continued)

| Equilibrium solubility of sertraline base and hydrochloride in selected vehicles | | |
|---|---|---|
| **Vehicle** | **Free base sol. in mgA/ml** | **Hydrochloride Sol in mgA/mL** |
| Olive oil | >50 | <0.01 (below detection limit) |
| Safflower oil | >50 | <0.01 (below detection limit) |
|  |  |  |

**HPLC Assay for sertraline:**

Reverse phase high performance liquid chromatography (HPLC) was used to evaluate sertraline concentration.

HPLC conditions:

Mobile phase: per liter of mobile phase: 270 mL of tetrahydrofuran, 230 mL of methanol and 400 mL of buffer. The buffer consisted of 25 mM triethylamine phosphate. It was prepared by adding 1.7 mL of phosphoric acid and 3.5 mL of tirethylamine to 1 liter of water. The pH of the final mobile phase was adjusted to an apparent pH value of 8.0± 0.1 with triethylamine.

Flow rate of mobile phase: 1.0 mU minute

Column:

Presaturator column: Waters Symmetry, C-18, 3.0x4.6 mm guard cartridge placed after the pump and before the autosampler.

Guard column: Waters Symmetry, C-18, 3.0x4.6 mm guard cartridge placed after the autosampler and before the analytical column.

Analytical column: Waters Symmetry, C-18, 250x4.6 mm

Detection: UV at 230 nm

Column Heater: 35°C

Injection volume: 20 μL

The response factor for the standard solution was used to calculate the concentration of sertraline in the sample.

$$\text{Response factor (RF)} = \frac{A_R \times DF}{W_R \times P}$$

where

$A_R$ =    area of peak in standard
$W_R$ =    weight of working standard
$P$ =    purity factor of working standard in decimal (e.g. 99.2% = 0.992)
$DF$ =    dilution factor

Example 5

Solutions of sertraline base are prepared in Capmul® -MCM at a concentration of 50 and 100 mgA/ml. The solutions are encapsulated in soft-gelatin at a fill volume of 0.5 ml, giving a unit dose of 25 and 50 mgA, respectively.

### Example 6

A solution of sertraline base is prepared in Capmul® -MCM at a concentration of 125 mgA/ml. The solution is encapsulated in soft-gelatin at a fill volume of 0.8 ml, giving a unit dose of 100 mgA.

### Example 7

Solutions of sertraline base are prepared in safflower, sesame, olive, or corn oil at a concentration of 50 and 100 mgA/ml. The solutions are encapsulated in soft-gelatin at a fill volume of 0.5 ml, giving a unit dose of 25 and 50 mgA, respectively.

### Example 8

Solutions of sertraline base are prepared in safflower, sesame, olive, or corn oil at a concentration of 200 mgA/ml. The solutions are encapsulated in soft-gelatin at a fill volume of 0.5 ml, giving a unit dose of 100 mgA.

### Example 9

Solutions of sertraline base are prepared in Polysorbate-80 at concentrations of 37.5 and 75 mgA/ml. The solutions are encapsulated in soft-gelatin at a fill volume of 0.67 ml, giving a unit dose of 25 and 50 mgA, resepctively.

### Example 10

Solutions of sertraline lactate are prepared in Capmul®-MCM at a concentration of 37.5 and 75 mgA/ml. The solutions are encapsulated in soft-gelatin at a fill volume of 0.67 ml, giving a unit dose of 25 and 50 mgA. respectively.

### Example 11

A solution of sertraline base is prepared in safflower oil/PEG-400 (80/20 v/v) at a concentration of 62.5 mgA/mf. The solution is encapsulated in soft gelatin at a fill volume of 0.8 ml to give a unit dose of 50 mgA. Likewise, a 31.25 mgA/ml solution is encapsulated to give a dose of 25 mgA.

### Example 12

A solution of sertraline base is prepared in safflower oil/Polysorbate-80 (80/20 v/v) at a concentration of 62.5 mgA/ml. The solution is encapsulated in soft gelatin at a fill volume of 0.8 ml to give a unit dose of 50 mgA. Likewise, a 31.25 mgA/ml solution is encapsulated to give a dose of 25 mgA.

### Example 13

A solution of sertraline base is prepared in Capmul® -MCM/Polysorbate-80 (80/20 v/v) at a concentration of 62.5 mgA/ml. The solution is encapsulated in soft gelatin at a fill volume of 0.8 ml to give a unit dose of 50 mgA. Likewise, a 31.25 mgA/ml solution is encapsulated to give a dose of 25 mgA.

### Example 14

A solution of sertraline base is prepared in Miglyol® -810/Polysorbate-80 (80/20 v/v) at a concentration of 62.5 mgA/ml. The solution is encapsulated in soft gelatin at a fill volume of 0.8 ml to give a unit dose of 50 mgA. Likewise, a 31.25 mgA/ml solution is encapsulated to give a dose of 25 mgA.

### Example 15

A solution of sertraline base is prepared in Capmul®-MCM/Miglyol®-810/Polysorbate-80 (60/20/20 v/v/v) at a concentration of 62.5 mgA/ml. The solution is encapsulated in soft gelatin at a fill volume of 0.8 ml to give a unit dose of 50 mgA. Likewise, a 31.25 mgA/ml solution is encapsulated to give a dose of 25 mgA.

Example 16

The sertratine solutions of Examples 5-15 are prepared at 20 mgA/ml, and 0.5 ml are encapsulated in soft gelatin capsules to give a dose of 10 mgA.

Example 17

The sertraline solutions of Examples 5-16 are encapsulated in hard gelatin capsules, and the connection seam between the two capsule halves is sealed with gelatin.

**Claims**

1. A dosage form comprising a gelatin-encapsulated composition comprising sertraline, or a pharmaceutically acceptable salt thereof, and a water-immiscible vehicle.

2. A dosage form as defined in claim 1, which decreases $T_{max}$ by at least 0.5 hr.

3. A dosage form as defined in claim 2, which decreases $T_{max}$ by at least one hour.

4. A dosage form as defined in claim 1, containing 10 to 300 mg of active sertraline equivalent to the free base.

5. A dosage form as defined in claim 4, containing 10 to 250 mg of active sertraline equivalent to the free base.

6. A dosage form as defined in claim 5, containing 10 to 100 mg of active sertraline equivalent to the free base.

7. A dosage form as defined in claim 1, wherein said vehicle comprises a mono-, di-, or triglyceride, or a mixture thereof.

8. A dosage form as defined in claim 7, wherein the acyl chain(s) of said mono-, di-, or triglyceride average 4-18 carbons in length.

9. A dosage form as defined in claim 8, wherein the acyl chain(s) of said mono-, di-, or triglyceride average 6-14 carbons in length.

10. A dosage form as defined in claim 1, wherein said vehicle is liquid at 37°C.

11. A dosage form as defined in claim 1, wherein said vehicle comprises a vegetable oil.

12. A dosage form as defined in claim 11, wherein said vegetable oil is selected from corn oil, peanut oil, sesame oil, olive oil, castor oil, coconut oil, cottonseed oil, soybean oil, or safflower oil.

13. A dosage form as defined in claim 1, wherein said vehicle comprises a surfactant or emulsifier.

14. A dosage form as defined in claim 13, wherein said surfactant or emulsifier is selected from polysorbate 80, nonylphenoxypolyoxyethylene, dioctyl sodium sulfosuccinate, PEG-6-glycerylmono-oleate, or PEG-6-glyceryllinoleate.

15. A dosage form as defined in claim 1, wherein said vehicle comprises a fatty acid.

16. A dosage form as defined in claim 15, wherein said fatty acid is selected from caprylic acid, capric acid, lauric acid, oleic acid, or linoleic acid.

17. A dosage form as in claim 1, wherein said dosage form comprises a liquid ester of a short chain alcohol and acid.

18. A dosage form as in claim 17, wherein said ester is selected from the propylene glycol esters of caprylic and/or capric acids.

**19.** A dosage form as In claim 1, wherein said vehicle additionally comprises an alcohol.

**20.** A dosage form as in daim 19, wherein said alcohol Is polyethyleneglycol, glycerin, ethanol, or propylene glycol.

**21.** A dosage form as defined in claim 1, which decreases precipitation of sertraline in a chloride ion containing use environment relative to a comparison dosage form made with a water-miscible vehicle.

**Patentansprüche**

**1.** Dosierungsform, die eine Sertralin oder ein pharmazeutisch akzeptables Salz desselben und ein mit Wasser nicht mischbares Vehikel umfassende gelatineverkapselte Zusammensetzung umfasst.

**2.** Dosierungsform gemäß der Definition in Anspruch 1, die $T_{max}$ um mindestens 0,5 h verringert.

**3.** Dosierungsform gemäß der Definition in Anspruch 2, die $T_{max}$ um mindestens 1 h verringert.

**4.** Dosierungsform gemäß der Definition in Anspruch 1, die 10 bis 300 mg von der freien Base äquivalentem aktivem Sertralin enthält.

**5.** Dosierungsform gemäß der Definition in Anspruch 4, die 10 bis 250 mg von der freien Base äquivalentem aktivem Sertralin enthält.

**6.** Dosierungsform gemäß der Definition in Anspruch 5, die 10 bis 100 mg von der freien Base äquivalentem aktivem Sertralin enthält.

**7.** Dosierungsform gemäß der Definition in Anspruch 1, wobei das Vehikel ein Mono-, Di- oder Triglycerid oder ein Gemisch derselben umfasst.

**8.** Dosierungsform gemäß der Definition in Anspruch 7, wobei die Acylkette bzw. Acylketten des Mono-, Di- oder Triglycerids eine durchschnittliche Länge von 4 - 18 Kohlenstoffen aufweisen.

**9.** Dosierungsform gemäß der Definition in Anspruch 8, wobei die Acylkette bzw. Acylketten des Mono-, Di- oder Triglycerids eine durchschnittliche Länge von 6 - 14 Kohlenstoffen aufweisen.

**10.** Dosierungsform gemäß der Definition in Anspruch 1, wobei das Vehikel bei 37 °C flüssig ist.

**11.** Dosierungsform gemäß der Definition in Anspruch 1, wobei das Vehikel ein pflanzliches Öl umfasst.

**12.** Dosierungsform gemäß der Definition in Anspruch 11, wobei das pflanzliche Öl aus Maisöl, Erdnussöl, Sesamöl, Olivenöl, Rizinusöl, Kokosöl, Baumwollsaatöl, Sojaöl oder Safloröl ausgewählt ist.

**13.** Dosierungsform gemäß der Definition in Anspruch 1, wobei das Vehikel ein Netzmittel oder einen Emulgator umfasst.

**14.** Dosierungsform gemäß der Definition in Anspruch 13, wobei das Netzmittel oder der Emulgator aus Polysorbat 80, Nonylphenoxypolyoxyethylen, Dioctylnatriumsulfosuccinat, PEG-6-glycerylmono-oleat oder PEG-6-glyceryllinoleat ausgewählt ist.

**15.** Dosierungsform gemäß der Definition in Anspruch 1, wobei das Vehikel eine Fettsäure umfasst.

**16.** Dosierungsform gemäß der Definition in Anspruch 15, wobei die Fettsäure aus n-Caprylsäure, n-Caprinsäure, Laurinsäure, Ölsäure oder Linoleinsäure ausgewählt ist.

**17.** Dosierungsform gemäß Anspruch 1, wobei die Dosierungsform einen flüssigen Ester eines kurzkettigen Alkohols und einer Säure umfasst.

**18.** Dosierungsform gemäß Anspruch 17, wobei der Ester aus den Propylenglykolestern von n-Capryl- und/oder n-Caprinsäuren ausgewählt ist.

**19.** Dosierungsform gemäß Anspruch 1, wobei das Vehikel zusätzlich einen Alkohol umfasst.

**20.** Dosierungsform gemäß Anspruch 19, wobei der Alkohol Polyethylenglykol, Glycerin, Ethanol oder Propylenglykol ist.

**21.** Dosierungsform gemäß der Definition in Anspruch 1, die die Ausfällung von Sertralin in einer Chloridionen enthaltenden Verwendungsumgebung in Bezug auf eine Vergleichsdosierungsform, die mit einem mit Wasser mischbaren Vehikel hergestellt wurde, verringert.

**Revendications**

**1.** Forme posologique comprenant une composition, encapsulée dans de la gélatine, comprenant de la sertraline, ou un de ses sels pharmaceutiquement acceptables, et un véhicule non miscible à l'eau.

**2.** Forme posologique suivant la revendication 1, qui présente une diminution de $T_{max}$ d'une valeur d'au moins 0,5 h.

**3.** Forme posologique suivant la revendication 2, qui présente une diminution de $T_{max}$ d'au moins une heure.

**4.** Forme posologique suivant la revendication 1, contenant 10 à 300 mg d'équivalent de sertraline par rapport à la base libre.

**5.** Forme posologique suivant la revendication 4, contenant 10 à 250 mg d'équivalent de sertraline actif par rapport à la base libre.

**6.** Forme posologique suivant la revendication 5, contenant 10 à 100 mg d'équivalent de sertraline actif par rapport à la base libre.

**7.** Forme posologique suivant la revendication 1, dans laquelle ledit véhicule comprend un mono-, di- ou triglycéride ou un de leurs mélanges.

**8.** Forme posologique suivant la revendication 7, dans laquelle la ou les chaînes acyle dudit mono-, di- ou triglycéride ont en moyenne une longueur de 4 à 18 atomes de carbone.

**9.** Forme posologique suivant la revendication 8, dans laquelle la ou les chaînes acyle dudit mono-, di- ou triglycéride ont en moyenne une longueur de 6 à 14 atomes de carbone.

**10.** Forme posologique suivant la revendication 1, dans laquelle ledit véhicule est liquide à 37°C.

**11.** Forme posologique suivant la revendication 1, dans laquelle ledit véhicule comprend une huile végétale.

**12.** Forme posologique suivant la revendication 11, dans laquelle ladite huile végétale est choisie entre l'huile de mais, l'huile d'arachide, l'huile de sésame, l'huile d'olive, l'huile de ricin, l'huile de copra, l'huile de graines de cotonnier, l'huile de soja et l'huile de carthame.

**13.** Forme posologique suivant la revendication 1, dans laquelle ledit véhicule comprend un agent tensioactif ou émulsionnant.

**14.** Forme posologique suivant la revendication 13, dans laquelle ledit agent tensioactif ou émulsionnant est choisi entre le polysorbate 80, le nonylphénoxypolyoéthylène, le dioctylsulfosuccinate de sodium, le monooléate de PEG-6-glycéryle ou le linoléate de PEG-6-glycéryle.

**15.** Forme posologique suivant la revendication 1, dans laquelle ledit véhicule comprend un acide gras.

**16.** Forme posologique suivant la revendication 15, dans laquelle ledit acide gras est choisi entre l'acide caprylique, l'acide caprique, l'acide laurique, l'acide oléique et l'acide linoléique.

**17.** Forme posologique suivant la revendication 1, qui comprend un ester liquide d'un alcool et d'un acide à chaîne courte.

**18.** Forme posologique suivant la revendication 17, dans laquelle ledit ester est choisi entre les esters de propylèneglycol d'acide caprylique et/ou d'acide caprique.

**19.** Forme posologique suivant la revendication 1, dans laquelle ledit véhicule comprend en outre un alcool.

**20.** Forme posologique suivant la revendication 19, dans laquelle ledit alcool est le polyéthylèneglycol, le glycérol, l'éthanol ou le propylèneglycol.

**21.** Forme posologique suivant la revendication 1, qui diminue la précipitation de la sertraline dans un environnement d'utilisation contenant des ions chlorure par rapport à une forme posologique comparative préparée avec un véhicule miscible à l'eau.